Europäisches Patentamt

European Patent Office

Office européen des brevets

(11) Veröffentlichungsnummer: **0 268 950**
**A2**

(12) **EUROPÄISCHE PATENTANMELDUNG**

(21) Anmeldenummer: 87116763.1

(51) Int. Cl.4: **A61K 7/40** , **A61K 7/48**

(22) Anmeldetag: 13.11.87

(30) Priorität: 25.11.86 DE 3640177

(43) Veröffentlichungstag der Anmeldung:
01.06.88 Patentblatt 88/22

(84) Benannte Vertragsstaaten:
**AT DE ES IT SE**

(71) Anmelder: **BAYER AG**
**Konzernverwaltung RP Patentabteilung**
**D-5090 Leverkusen 1 Bayerwerk(DE)**

(72) Erfinder: **Steinbach, Hans-Horst, Dr.**
**Im Birkelshof 1**
**D-5060 Bergisch Gladbach 2(DE)**
Erfinder: **Bernd, Wittmann**
**Scheurenerstrasse 80**
**D-5068 Odenthal(DE)**

(54) **Hautschützende bzw. hautpflegende Zusammensetzung.**

(57) Die vorliegende Erfindung betrifft eine hautpflegende bzw. hautschützende Zusammensetzung mit einem Gehalt an hochviskosen Polydimethylsiloxanen, die gegebenenfalls end-und/oder mittelständige OH-, Vinyl-, Phenyl-oder Alkylgruppen mit 2 bis 6 C-Atomen aufweisen, mit einer Viskosität von $10^6$ bis $5.10^7$ mPa.s und leicht flüchtigen oder zyklischen Siloxanen.

EP 0 268 950 A2

## Hautschützende bzw. hautpflegende Zusammensetzung

Die vorliegende Anmeldung betrifft eine Zusammensetzung, die als Hautschutz, insbesondere für die Hände geeignet ist.

Hautschutzmittel - speziell als Schutz der Hände vor Verschmutzung und Chemikalien sowie mechanischer Beanspruchung - sind in unterschiedlichen Zusammensetzungen bekannt.

Zumeist basieren derartige Cremes oder Lotions auf Polydimethylsiloxan, auf Abkömmlingen oder Copolymeren dieses Siliconpolymers, wobei zum Teil wasserlösliche Harze, Acrylate, Carboxyvinylpolymere, Vinylester, Vinylacetat, Vinylpyrrolidon und Wachse als Filmbildner bzw. Verdicker eingesetzt werden.

Als pflegende Komponente wird außer Siliconen (Polydimethylsiloxane und flüchtige Silicone) Glycerin verwendet. Als Emulgier-/Homogenisiermittel dienen Tenside.

Die im Markt erhältlichen diesbezüglichen Schutzcremes bzw. Lotions werden durch die erfindungsgemäßen Maßnahmen in bezug auf einen ausreichend stabilen Schutzfilm verbessert. Voraussetzung für die richtige Funktion des Schutzfilms sind dessen ausreichende Verteilbarkeit, Filmdicke, Gleiteigenschaften und Abriebstabilität. Es hat sich gezeigt, daß mit zunehmender Viskosität des Polydimethylsiloxans der Hautschutz verbessert, die Einarbeitung aber verschlechtert wird. Deswegen hat sich der Einsatz von Polydimethylsiloxanen mit Viskositäten zwischen 10 und 1000 mPa.s durchgesetzt. Es wurde nun gefunden, daß diese Eigenschaften durch die hier beschriebenen Wirkstoffkombinationen, bei denen es sich um Zubereitungen handelt, deutlich verbessert werden.

Innerhalb des reichhaltigen Angebots filmbildender Silicone können wesentliche Verbesserungen der Filmstabilität erzielt werden, indem man Lösungen sehr hochviskoser Polydimethylsiloxane in flüchtigen niederpolymeren linearen oder zyklischen Siloxanen auflöst und diese Lösungen zu 10-40 %, vorzugsweise in handelsübliche Handcremes (O/W-System) einemulgiert bzw. fein verteilt einarbeitet. Die Kombination einer solchen Lösung verschiedener Polysiloxane hat den Vorteil leichter Verteilbarkeit auf der Haut. Nach dem Abdampfen der flüchtigen Verbindungen erhält man einen Schutzfilm mit guter Haftung, geringen Abtragssowie guten Trenneigenschaften. Der aus hochviskosem Polydimethylsiloxan bestehende Schutzfilm ist außerdem wasser-und schmutzabweisend.

Der Gegenstand der vorliegenden Erfindung ist somit eine hautpflegende bzw. hautschützende Zusammensetzung, gekennzeichnet durch einen Gehalt an hochviskosen Polydimethylsiloxanen, die gegebenenfalls end-und/oder mittelständige OH-, Vinyl-, Phenyl-oder Alkylgruppen mit 2 bis 6 C-Atomen aufweisen, mit einer Viskosität von $10^6$ bis $5.10^7$ mPa.s und leicht flüchtigen linearen oder zyklischen Siloxanen.

Die Gehalte an Methyl-vinyl-siloxy-Gruppen liegen etwa zwischen 200 ppm und 20 Gew.-%, die Gehalte an OH-$Me_2$-$SiO_{1/2}$ liegen zwischen 100 und 1000 ppm. Das Verhältnis Phenylgruppen zu Methylgruppen liegt im Bereich von 1:2,2 bis 1:5,1.

Diese hochviskosen Polydimethylsiloxane können in üblichen Mischaggregaten wie Schüttelmaschine, Dissolver, Planetenmischer und Kneter in flüchtigen zyklischen oder linearen Dimethylsiloxanen aufgelöst werden. Als physiologisch unbedenkliche niedrigviskose Siloxanlösungsmittel haben sich besonders Hexamethyldisiloxan, Octamethylcyclotetrasiloxan und Decamethylcyclopentasiloxan bewährt. 10 bis 40 %ige Lösungen dieser hochviskosen Polydimethylsiloxane in flüchtigen zyklischen oder linearen Dimethylsiloxanen zeigen Viskositäten von 300 bis 100.000 mPa.s und sind in handelsübliche Handcremes leicht einarbeitbar. Die Konzentration der Wirkstoffkombination in handelsüblichen Handcremes kann zwischen 10 und 50 % liegen. Eine Zugabe von etwa 40 % hat sich für die Herstellung eines "flüssigen Handschuhs" als recht günstig herausgestellt.

Entsprechende erfindungsgemäße Zusammensetzungen haben somit einen Gehalt an hochviskosem Polydimethylsiloxan von etwa 5 bis 20 Gew.-%, bevorzugt 8 bis 15 Gew.-%, und einen Gehalt von 10 bis 50 Gew.-%, bevorzugt 20 bis 40 Gew.-% an leicht flüchtigen Siloxanen - bezogen auf die gesamte Zusammensetzung.

Hervorzuheben ist, daß nach dem Waschen der mit dem "flüssigen Handschuh" geschützten Haut ein dünner pflegender Schutzfilm auf der Haut zurückbleibt, der die Haut auch nachträglich noch der Angriffen durch z.B. Öl und Chemikalien - schützt.

Die Herstellung der erfindungsgemäß einzusetzenden Wirkstoffkombination kann auf Basis der anschließend aufgeführten Rezepturen wie folgt durchgeführt werden:

Die entsprechende Menge des Polymers wird in einem Kneter, Planetenmischer oder Dissolver dem vorgelegten flüchtigen Siloxan zugegeben. Da die eingesetzten flüchtigen Siloxane brennbar sind, empfiehlt sich die Überleitung eines Inertgases (z.B. Stickstoff) während des gesamten Lösungszeitraumes. Wegen der hohen Flüchtigkeit sollten Rührgefäße bzw. Kneter möglichst verschlossen sein und das Inertgas mit geringer Strömungsgeschwindigkeit übergeleitet werden.

Typische Rezepturen, wie sie erfindungsgemäß eingesetzt werden, sind etwa wie folgt (%-Angaben sind Gew.-%):

Der Gehalt des in den Rezepturen genannten hochviskosen Polydimethylsiloxans an Methyl-vinylgruppen beträgt 0,1 Gew.-%.

1) 20 % vinylgruppenhaltiges, hochviskoses Polydimethylsiloxan (Viskosität ca. 18.000.000 mPa.s)
80 % Octamethylcyclotetrasiloxan

2) 20 % vinylgruppenhaltiges, hochviskoses Polydimethylsiloxan (Viskosität ca. 18.000.000 mPa.s)
80 % Decamethylcyclopentasiloxan

3) 20 % vinylgruppenhaltiges, hochviskoses Polydimethylsiloxan (Viskosität ca. 19.000.000 mPa.s).
80 % Hexamethyldisiloxan

4) 25 % vinylgruppenhaltiges, hochviskoses Polydimethylsiloxan (Viskosität ca. 18.500.000 mPa.s)
75 % Octamethylcyclotetrasiloxan

5) 25 % vinylgruppenhaltiges, hochviskoses Polydimethylsiloxan (Viskosität ca. 31.200.000 mPa.s)
75 % Decamethylcyclopentasiloxan

6) 25 % vinylgruppenhaltiges, hochviskoses Polydimethylsiloxan (Viskosität ca. 18.000.000 mPa.s)
75 % Hexamethyldisiloxan

7) 30 % vinylgruppenhaltiges, hochviskoses Polydimethylsiloxan (Viskosität ca. 45.100.000 mPa.s)
70 % Octamethylcyclotetrasiloxan

8) 30 % vinylgruppenhaltiges, hochviskoses Polydimethylsiloxan (Viskosität ca. 18.800.000 mPa.s)
70 % Decamethylcyclopentasiloxan

9) 30 % vinylgruppenhaltiges, hochviskoses Polydimethylsiloxan (Viskosität ca. 39.600.000 mPa.s)
70 % Hexamethyldisiloxan

10) 40 % vinylgruppenhaltiges, hochviskoses Polydimethylsiloxan (Viskosität ca. 27.100.000 mPa.s)
60 % Octamethylcyclotetrasiloxan

11) 40 % vinylgruppenhaltiges, hochviskoses Polydimethylsiloxan (Viskosität ca. 48.000.000 mPa.s)
60 % Decamethylcyclopentasiloxan

12) 40 % vinylgruppenhaltiges, hochviskoses Polydimethylsiloxan (Viskosität ca. 18.500.000 mPa.s)
60 % Hexamethyldisiloxan

13) 20 % hochviskoses, endständige OH-Gruppen enthaltendes Polydimethylsiloxan (Viskosität ca. 14.000.000 mPa.s)
80 % Octamethylcyclotetrasiloxan

14) 20 % hochviskoses, endständige OH-Gruppen enthaltendes Polydimethylsiloxan (Viskosität ca. 30.500.000 mPa.s)
80 % Decamethylcyclopentasiloxan

15) 20 % hochviskoses, endständige OH-Gruppen enthaltendes Polydimethylsiloxan (Viskosität ca. 25.000.000 mPa.s)
80 % Hexamethyldisiloxan

16) 25 % hochviskoses, endständige OH-Gruppen enthaltendes Polydimethylsiloxan (Viskosität ca. 41.700.000 mPa.s)
75 % Octamethylcyclotetrasiloxan

17) 25 % hochviskoses, endständige OH-Gruppen enthaltendes Polydimethylsiloxan (Viskosität ca. 16.000.000 mPa.s)
75 % Decamethylcyclopentasiloxan

18) 25 % hochviskoses, endständige OH-Gruppen enthaltendes Polydimethylsiloxan (Viskosität ca. 16.000.000 mPa.s)
75 % Hexamethyldisiloxan

19) 30 % hochviskoses, endständige OH-Gruppen enthaltendes Polydimethylsiloxan (Viskosität ca. 35.500.000 mPa.s)
70 % Octamethylcyclotetrasiloxan

20) 30 % hochviskoses, endständige OH-Gruppen enthaltendes Polydimethylsiloxan (Viskosität ca. 27.000.000 mPa.s)
70 % Decamethylcyclopentasiloxan

21) 30 % hochviskoses, endständige OH-Gruppen enthaltendes Polydimethylsiloxan (Viskosität ca. 17.000.000 mPa.s)
70 % Hexamethyldisiloxan

22) 40 % hochviskoses, endständige OH-Gruppen enthaltendes Polydimethylsiloxan (Viskosität ca. 37.100.000 mPa.s)
60 % Octamethylcyclotetrasiloxan

23) 40 % hochviskoses, endständige OH-Gruppen enthaltendes Polydimethylsiloxan (Viskosität ca. 15.000.000 mPa.s)
60 % Decamethylcyclopentasiloxan

24) 40 % hochviskoses, endständige OH-Gruppen enthaltendes Polydimethylsiloxan (Viskosität ca. 46.500.000 mPa.s)
60 % Hexamethyldisiloxan

Sehr gute Einarbeitung der Wirkstoffkombination ist gegeben bei Viskositäten im Bereich von 10.000 bis 20.000 mPa.s. Bei der Kombination von 25 % des hochviskosen, endständige OH-Gruppen enthaltenden Polydimethylsiloxans mit 75 % flüchtigem Siloxan erhält man ein Optimum an gewünschten Eigenschaften. Höhere Konzentrationen führen zum gleichen Erscheinungsbild wie bei der Kombination von 40 % vinylgruppenhaltigem Polydimethylsiloxan mit 60 % flüchtigen Siloxanen. Von den diversen Lösungen der OH-Gruppen bzw. Vinylgruppen enthaltenden Polymeren in flüchtigen Siloxanen wurden folgende Viskositäten gemessen:

| % Vinylpolymer | % flüchtiges Siloxan | Viskosität (mPa.s) |
|---|---|---|
| 10 | 90 | 391 |
| 20 | 80 | 5.100 |
| 25 | 75 | 14.600 |
| 30 | 70 | 26.700 |
| 40 | 60 | 92.400 |

| % OH-Polymer | % flüchtiges Siloxan | Viskosität (mPa.s) |
|---|---|---|
| 10 | 90 | 631 |
| 20 | 80 | 14.750 |
| 25 | 75 | 28.600 |
| 30 | 70 | 45.530 |
| 40 | 60 | 38.800 |

Die erfindungsgemäßen Zusammensetzungen können in beliebige Cremes bzw. Lotionen eingearbeitet werden. Besonders geeignet sind Handcreme-Zusammensetzungen, doch ist der Erfindungsgegenstand keineswegs darauf beschränkt.

Die Erfindung soll anhand der folgenden Beispiele noch näher erläutert werden:

Beispiel 1

25 Gew.-% einer Wirkstoffkombination bestehend aus 25 Gew.-% vinylgruppenhaltigem Polydimethylsiloxan (Viskosität ca. 25.000.000 mPa.s) und 75 Gew.-% Octamethylcyclotetrasiloxan wurden zu 75 Gew.-% einer Standardhandcreme zugemischt.

Die Standardhandcreme wurde hergestellt aus:
a) 8,30 Gew.-% Cetylstearylalkohol
2.00 Gew.-% Cetylstearylalkohol mit ca. 10 Mol Ä.O.
5,00 Gew.-% Fettsäuremono-und -diglycerid
3,00 Gew.-% Ölsäuredecylester
3,00 Gew.-% Polydimethylsiloxan (350 mPa.s)
0,05 Gew.-% p-Hydroxybenzoesäurepropylester
b) 51,90 Gew.-% Wasser, dest.
0,15 Gew.-% p-Hydroxybenzoesäuremethylester
25,00 Gew.-% Glycerin DAB 7
1,00 Gew.-% Natriumcetylstearylsulfat
0,20 Gew.-% 5-Ureidohydantoin
0,40 Gew.-% Parfümöl

Beispiel 2

40 Gew.-% einer Wirkstoffkombination bestehend aus 25 Gew.-% vinylgruppenhaltigem Polydimethylsiloxan (Vinylgruppengehalt ca. 900 ppm) mit einer Viskosität von ca. 21.000.000 mPa.s und 75 Gew.-% Octamethylcyclotetrasiloxan wurden zu 60 Gew.-% einer handelsüblichen Handcreme (Stokolan® der Fa. Stockhausen) zugegeben.

Diese Versuchsrezepturen führten zu guten Ergebnissen hinsichtlich der Einarbeitbarkeit der ausgewählten Wirkstoffkombination sowie der Auftragbarkeit und der Funktionseigenschaften.

Als Optimum erwies sich eine Mischung von 40 % einer Wirkstoffkombination bestehend aus 25 % eines endständige OH-Gruppen enthaltenden Polydimethylsiloxans und 75 % eines flüchtigen Siloxans (Octamethylcyclotetrasilocan) und 60 % einer handelsüblichen Handcreme.

Es hat sich gezeigt, daß mit steigender Viskosität des eingesetzten Polymers die Haltbarkeit des Schutzfilms nach dem Reinigen der Hände und die schmutzabweisenden Eigenschaften verbessert werden.

Je nach der zu verrichtenden Tätigkeit sollten die Hände zwischen Arbeitsbeginn und den Pausen bzw. dem Arbeitsende gewaschen und erneut "flüssiger Handschuh" aufgetragen werden und zwar, indem man eine ausreichende Menge in hergebrachter Weise auf den Innen-und Außenflächen der Hände gut verteilt. Zu achten ist darauf, daß auch unter die Fingernägel reichlich Creme gelangt.

Das Waschen der Hände kann mit einer handelsüblichen Seife oder flüssigen Seife erfolgen. Die Anwendung von Spezialseife oder Handwaschpaste, die der Haut bekanntlich Fett entziehen und sie rissig und spröde machen, entfällt.

Die Hautschutzwirkung wurde gegen Isopropanol im Bereich der klinischen Händedesinfektion geprüft. Dabei wurde in einem orientierenden Test die Abwaschbarkeit von übercremten Filzstiftmarkierungen auf der Haut geprüft. Nach mehrmaligem Händewaschen zeigten die mit dem beschriebenen flüssigen Handschuh behandelten Hände ins Auge springende Unterschiede gegenüber den normalen handelsüblichen Handcremes.

**Ansprüche**

1. Hautpflegende bzw. hautschützende Zusammensetzung, gekennzeichnet durch einen Gehalt an hochviskosen Polydimethylsiloxanen, die gegebenenfalls end-und/oder mittelständige OH-, Vinyl-, Phenyl-oder Alkylgruppen mit 2 bis 6 C-Atomen aufweisen, mit einer Viskosität von $10^6$ bis $5.10^7$ mPa.s und leicht flüchtigen linearen oder zyklischen Siloxanen.

2. Zusammensetzung gemäß Anspruch 2, dadurch gekennzeichnet, daß der Gehalt an hochviskosen Polydimethylsiloxanen 5 bis 20 Gew.-% und der Gehalt an leicht flüchtigen Siloxanen 10 bis 50 Gew.-% - bezogen auf die gesamte Zusammensetzung - beträgt.